# EUROPEAN PATENT SPECIFICATION

(11) **EP 0 836 496 B1**
(45) Date of publication and mention of the grant of the patent: **24.10.2001**
(21) Application number: 96921198.6
(22) Date of filing: 19.06.1996
(51) Int. Cl.: A61M 15/02, A61M 15/00

(54) **A DEVICE FOR AN INHALER**
VORRICHTUNG FÜR INHALATOR
DISPOSITIF POUR INHALATEUR

(30) Priority: 21.06.1995 SE 9502262
(43) Date of publication of application: 22.04.1998
(73) Proprietor: Microdrug AG, 6052 Hergiswil NM (CH)
(72) Inventor: Nilsson, Lars Gunnar, 731 30 Köping (SE)
(74) Representative: Mrazek, Werner
(86) International application number: PCT/SE96/00807
(87) International publication number: WO 97/00704

(56) References cited:
- WO-A-92/09322
- WO-A-92/15353
- WO-A-94/19042

## Description

### Field of the invention

The present invention relates to a device for electrical dosing of pure substances, or preparations consisting of chemical and biological substances mainly used as drugs, whereby the substances to be dosed first of all are in the form of dry powders, but may also be in the form of small drops or aerosols.

### Background of the invention

The dosing of drugs are carried out in a number of different ways in the medical service. In the medical service there are today for dry powders and aerosols a number of different inhalers in use, which all have as a common object to supply a substance to the lung through the patients own inspiration. The substances or mixtures of substances today used in the medical service are all developed to be supplied by means of a deep breath, which means high local velocities, which in turn means that a certain amount of the substance will stick to the airways and to the inhaler. The amount of substance in this way sticking also varies from individual to individual due to the individual way of breathing. This low efficiency and low dosing precision either means an increased risk for side effects if a too large dose is supplied, or risk for low effect if a too small dose is supplied. Thus, it is of the utmost importance that the dosing can be coordinated with the inspiration when using inhalers.

A method of improving the efficiency of an inhaler is disclosed in two patent documents, WO-9419042 and WO-9215353. The method means that the substance (powder or aerosol) is being electrically charged after the dosing and the distribution of the substance to the lung is in this way improved. The static electric charging of the substance is said to imply a more even distribution of the substance in the air stream at inspiration and therefore not sticking on to the inhaler or the airways to the same extent. The patent discloses a normal mechanical dosing.

The inhalers of today, the usage of which increase strongly, accordingly do have a number of disadvantages which after all limit their efficiency and usage. The following list of items exemplifies some of these disadvantages:
· A varying concentration of substance in the inhaled air.
· A very large proportion of the substance sticks on to the inhaler and/or the airways.
· Deviations in the inspiratory process can result in great deviations in the dose emitted to the lung.
· Today, only a small number of substances can be used in inhalers according to prior art.
· The amount of substance emitted to the lung can vary from patient to patient and between different dosing occasions.
· Inhalers according to prior art are generally not meant for repeated reuse.

### Description of the invention

The present invention relates to a device for electrical dosing of pure substances or preparations consisting of chemical and biological substances, which substances are mainly used as medicaments. The substances to be dosed are mainly in the form of dry powder, but may also be in the form of droplets or aerosols.

In particular, the present invention is meant to be applied at the construction of a new continuous inhaler, which thereby gives a number of advantages in comparison to inhalers according to prior art which are found on the market today.

The invention according to the independent claim 1 relates to a device in a continuous inhaler with a capacity of dosing a great number of different substances. With continuous inhaler is meant an equipment allowing a substance to be supplied continuously or by a repeated number of small doses with such a frequency that the result from the inhaler can be characterized as continuous. Dosing is connected to the air flow and the concentration and amount is controlled during the inspiratory process. This means a more even supply of substance and thereby a possibility to increase the amount of substance to the lung compared to the inhalers existing today.

The inhaler thus created is suitably equipped so as to be programmable to be directly flexible to the special preconditions of every patient with respect to inspiratory characteristics, need of substance, number of doses and dosing intervals. The new inhaler is first of all meant to be portable, but may also be stationary.

The dependent claims 2 to 13 relate to different embodiments of the present invention.

### Description of the drawings

The invention will be described in terms of a preferred and illuminating embodiment taking reference to the appended drawings where like reference symbols denote like or corresponding element, where:
- Fig. 1: shows an outline sketch of the function of an inhaler in accordance with the present invention,
- Fig. 2: shows a side view of an illuminating embodiment of the inhaler constructed in accordance with figure 1, and
- Fig. 3: shows the inhaler according to figure 2 in a view from above.

### An illustrative embodiment

### 1. Technical description

The substances which are to be dosed are mainly in the form of dry powder, but may also be in form of small drops and aerosols. Henceforth, in connection with the description of an illustrative embodiment powder will be used as a common name for all types of substances or preparations of substances which are to be dosed.

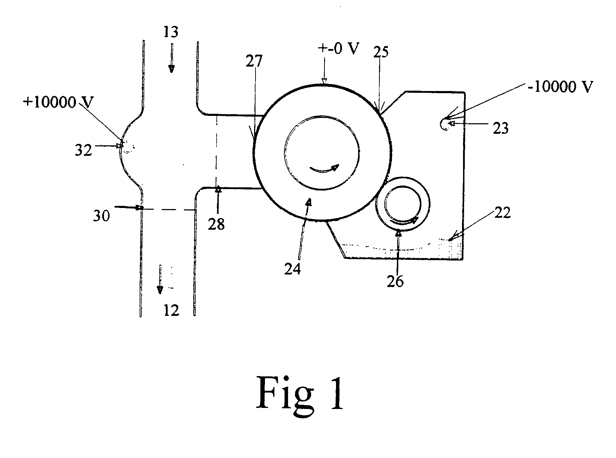

In figure 1 an outline sketch illustrating the invention in a simple way is shown. To the right in the sketch is provided a space 22 for storing the powder. In the same space an electric field is generated by means of an electrode device 23, which is given a suitable electric potential. The object of the electric field is the static charging of the powder particles. Said particles are then attracted to and sticking on to a rotating dosing drum 24 having the electric potential of approximately +/- 0 V. During the rotation superfluous powder is removed by means of a smoothing device 25. When the drum has completed half a revolution the powder will be exposed to a second electric field generated by a second electrode device 32 with a suitable opposite potential, whereby the powder is loosened and moved away from the drum 27 to an air stream 13, which is obtained from an inspiration, and is then mixed with said air stream and further transported out through the inhaler nozzle 12. On its way the powder passes an electronic grid 28 for the regulation of the dosing and a deionizing zone 30. The correct dose in form of a homogenous air/powder mixture then passes out through the mouth piece or nozzle 12.

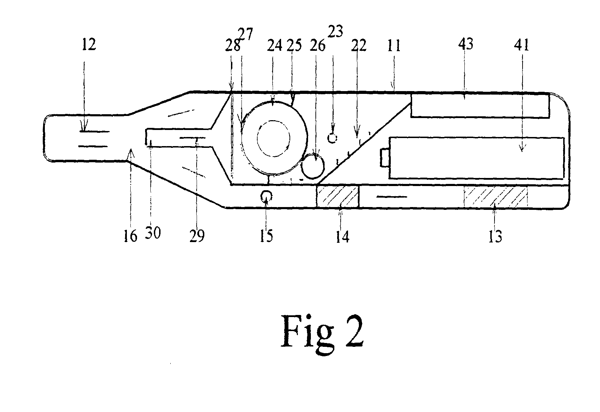

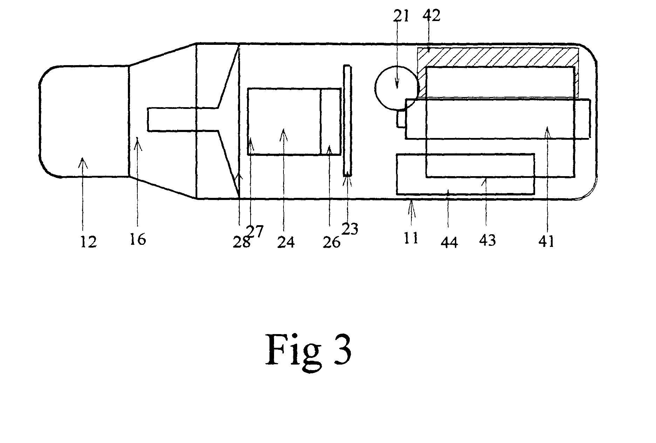

In figures 2 and 3 an illustrative embodiment of an inhaler in accordance with the present invention is shown. The inhaler has a cover 11, which is completely closed in order to prevent dust and moist to penetrate. When choosing the material for the manufacturing of the cover 11 and other mechanical parts the electric properties of the material is suitably devoted special attention in order to prevent the accumulation of powder in the wrong places. Also the cover 11 is preferably constructed with such a design that the least possible amount of powder can stick. The size of the inhaler is thus adapted that it can be carried in for example the pocket of a jacket. Inspiration is done through a nozzle 12 in the front portion of the inhaler and the air is at inspiration sucked in through a filter 13 on the underside of the inhaler. During the passage through the inhaler another filtration 14 and the measurement of the air velocity 15 takes place before the air is mixed with powder in the diffusor 16 in front of the nozzle 12.

The powder, which is stored in a cartridge, which may also contain a drying agent to ensure a correct level of humidity, is supplied to the inhaler through an opening 21 (see figure 3) on top of the inhaler. The opening is hermetically shut after the charging. The powder cartridge is preferably designed such that it automatically opens at the loading so that the powder is discharged in the storing space 22 of the inhaler and that also the closing takes place in the same operation. The amount of powder is adapted to the needs of the patient and can normally cover for example a complete period of treatment. In the storing space 22 of the powder there is an electrode 23, which when the inhaler is to be used is charged to a suitable voltage and then generates a field which in turn charges the powder particles to a suitable electrostatic potential. In the same space, further, a rotating dosing drum 24 with a different potential is provided, to which the powder particles will be attracted and stick. In order to obtain a smooth layer of powder on the cylinder a smoothing device 25 is provided. For cleaning the drum before the surface is coated with powder, a cleaning device 26, e.g., in the form of a rotating brush, is provided. When the dosing drum 24 rotates and the smoothed powder comes over to the other side 27 of the drum the powder will be influenced by a second electric field of an opposite potential, which thereby loosens the powder and transports it in the direction away from the drum. On the way to the nozzle 12 the powder passes through an electric grid 28 which regulates the amount of powder emitted from the dosing drum 27 and subsequently through a pipe containing a powder accelerator 29 and possibly a deionizing zone 30. After this the powder mixes with the inhaled air in a diffusor 16.

All the regulation, monitoring and memory functions are handled by a microprocessor 42 which is connected to a function indicator 43. Prior to the usage of the inhaler in order to provide correct dosing as well as providing correct control of the different functions of the inhaler the microprocessor is programmed through an external connection in accordance with prior art, so that an optimum result is to be achieved.

To supply the inhaler with power a rechargeable battery 41 is provided, which by means of an external switch on the cover of the inhaler can be switched on and off. For the driving of the mechanical equipment a motor 44 is supplied, which is coupled, among other things, to the driving means of the dosing drum in a way which is known in the art.

### 2. A mode of operation.

The function of the inhaler is based on dosing by means of the electric charging of a powder in a way which is described in the literature concerning modern printers and copy machines. The principle is illustrated in figure 1 and can briefly be described in the following way.

The powder 22 is statically charged by means in an electric field which is generated by an electrode device 23 with a suitable voltage. The powder will then be attracted to a device, in this case a rotating dosing drum 24, which has a potential of approximately +/- 0 V. The drum 24 will in this way continuously be coated with a thin layer of powder. On the opposite side 27 of the drum a suitable amount of powder is loosened by means of an electrode device 32 with an suitable opposite potential. The in this way dosed amount of powder is then drawn away and mixed by means of the air stream resulting from the inspiration, and continues through the inhaler and down into the patients lung system. The amount of powder emitted from the dosing position is regulated in many ways, for example by means of an electronic grid 28. If necessary, deionizing 30 takes place before the air/powder mixture leaves the inhaler 12. The loosening of a powder can be accomplished either intermittently in small doses which through the volume coming out from the inhaler will be understood as continuous, or through a truly continuous process where the dose is continuously emitted.

The demands on the electric properties of the powder are specific in the same way as when powder is dosed in a printer. To obtain the correct electric properties it is sometimes necessary to make some sort of preparation of the powder prior to usage. This preparation could for example be a micronisation of the powder to the correct size, preparations together with carriers having the right electric properties and or a surface treatment of the powder. An adaptation to the properties of the powder can also be done through choosing a suitable method for the electrostatic charging of the powder. Examples of different types of charging procedures are coronacharging, tribocharging and inductive charging. The charging procedure is connected to the microprocessor of the inhaler which is thereby programmed in a way known in the art in order to achieve an optimum result.

The amount of powder emitted is through the microprocessor connected to the air velocity through the inhaler in order to provide an as even as possible dose throughout the dosing process. This is advantageous since it means less powder deposits in the mouth and in other parts of the airways resulting from this procedure. The amount of air is measured by means of an airflow gauge 15 and the amount of powder is measured and dosed electrically by means of the electronic grid 28. The micro processor 42 stores and processes the results from the measurements throughout the dosing process in order to secure a correct emitted dose from the inhaler. By means of a function indicator 43 the inhaler warns if the inhalation should be incorrect, and for example too low a dose has been emitted. If a correct dose is emitted the inhaler gives a clearance and verifies and stores the emitted total dose.

After being emitted from the dosing equipment, depending on its properties, the powder may need some after-treatment as for example deionization 30, but also deagglomeration, separation of carriers and so forth, which is then carried out in the dose treatment member 29 of the inhaler, located in the flow direction subsequent to the dose emittance means. The dose treatment member is suitably equipped in accordance with the demands set by each powder.

Subsequent to being after-treated in the dose treatment member 29, 30 the powder is emitted through the nozzle 12 of the inhaler into the mouth of the patient for further transportation down into the lung. The nozzle 12 is designed so as to give the least possible substance deposit on teeth and in the oral cavity as well as achieving the least possible difference in air velocity between the inhaler and the oral cavity in order to minimize the ejector effect and thereby minimizing the risk for the substance being "shot" at the mucous membrane in the oral cavity. The nozzle 12 can be designed in alternatives ways to suit other types of administration of powder. In order to avoid the patient and the inhaler having different electric potentials at the inhalation, the nozzle of the inhaler is provided with an electrically conductive portion which is in contact with the patients mouth at the inhalation. In the case of different potentials the risk of powder deposits in the oral cavity or the upper airways increases.

Thus, the purpose of the inhaler according to the present invention is that no extra heavy breath shall be needed when inhaling but only, basically, a continuously normally calm but suitably deep breath. Inhalation of the at the time preferred total dose can, if necessary, thereby also take place by means of a number of subsequent breaths whereby the inhaler in accordance with the invention automatically regulates the dispensing of a correct amount of powder.

When the switch of the inhaler is put in the "on" position it automatically enters the stand-by position. The inhaler is then put to the mouth and when the inspiration starts the air flow sensor gives a signal to the micro processor, which immediately starts the dosing of the powder. Then, the micro processor controls the dosing process throughout the inspiration and makes sure that the correct dose has been emitted. When the patient has switched off the inhaler after usage, it is shut by for example placing a tight hood over the air intake as well as over the nozzle, thereby protecting the container of the substance, as well as all other moist sensitive details, from moist. The inhaler can therefore be brought in for example the pocket of a jacket or in a purse. The inhaler, which must always be personal, can thereby give the physician a possibility of following up the usage of the inhaler and the doses taken by the patient.

The micro processor is preferably programmed by means of one or more sample inspirations done by the patient, the characteristics of which is registered by and stored in the micro processor. This programming means that the dosing can be completely adapted to the inspiratory characteristics of every patient.

The regeneration of the inhaler with new powder is achieved by the insertion of a new powder cartridge in the inhaler and the resetting of the micro processor 42 of the inhaler. The battery 41 of the inhaler is in the preferred embodiment in itself an exchangeable but also rechargeable battery, which at suitable occasions is connected for recharging by means of a standard recharging device well known in the art and commercially accessible to the public.

## Claims

1. A device for an inhaler comprising a power source for the creation of suitable electric potentials for the administration of preferably powder chemical and biological substances, **characterized in that** the dosing is achieved by means of an electrostatic charging of the substance via a first field generating member (23) whereby the substance is transmitted to a transmission member, which attracts said substance and from which member the substance is then emitted by means of the influence of another electric field generated by a second field generating member (32).

2. The device according to claim 1, **characterized by** the transmission member, which transmission member transmits the substance to be dosed, consisting of a dosing drum (24), which during rotation by means of an built-in motor (26) will emit the substance by means of the influence from the additional electric field generated by the second field generating member (32).

3. The device according to claim 1 or 2, **characterized in that** the substance is emitted from the dosing drum (24) in the device by means of a combination of the additional electric field and the air stream resulting from the inspiration.

4. The device according to claim 1 to 2, **characterized in that** thereby created inhaler is additionally inspiration controlled, whereby no substance is emitted from the dosing drum (24) until an air stream has been detected by means of an air flow gauge (15) .

5. The device according to claim 1 or 2, **characterized in that** substance is dosed and measured by means of an electronic grid (28) while at the same time the amount of air is being measured by means of the air flow gauge (15), which provides a flexible and with high precision measurable dosing of the powder and enabling a high flexibility regarding the inspiration process of the patient.

6. The device according to claim 1 or 2, **characterized by** the created inhaler being furthermore provided with a treatment means consisting of an accelerator member (29), a deionizing member (30) and a diffusor member (16), which are thereby included in a patient friendly nozzle member.

7. The device according to claim 1 or 2, **characterized by** the created inhaler being provided with a micro processor (42) which can be programmed for the control of dosing, flows, time, number of doses, limits, security codes and so on, which furthermore enables an advanced follow up of the patient usage of the substance or substances during a period of time signaling for example overdosing, faulty usage or trends.

8. The device according to claim 1 or 2, **characterized in that** the created inhaler is provided with a function indicator (43) which by means of, for example, sound, light signals, and plain text warns in the case of risk for overdosing or faulty usage and also giving clearance in case of a correctly emitted and by the patient received dose.

9. The device according to claim 1 or 2, **characterized by** the inhaler being disposed so as to enable its receiving of a possible cartridge for the loading of the inhaler with the desired substance, the inhaler thereby being rendered easily chargeable and also rechargeable with one or more substances.

10. The device according to claim 1 or 2, **characterized in that** the powder substance is electrostatically charged by means of tribocharging or inductive charging to a suitable potential when this is better suited for the characteristics of the used powder.

11. The device according to claim 1 or 2, **characterized by** the inhaler being electrically connected with the person performing the inspiration thereby decreasing the risk of powder depositing in the mouth and in the upper airways.

12. The device according to claim 1 or 2, **characterized in that** the powder chemical and biological substances which are dosed are thus prepared that their electrical characteristics are suited for the electric dosing.

13. The device according to claim 1 or 2, **characterized in that** the electrical characteristics of the powder are optimized by the addition of carriers, or by surface treatment of the powder, and **in that** a correct particle size and particle structure is used.

## Patentansprüche

1. Vorrichtung für ein Inhalationsgerät, umfassend eine Energiequelle zur Erzeugung geeigneter elektrischer Potentiale zur Verabreichung vorzugsweise pulvriger chemischer und biologischer Substanzen, **dadurch gekennzeichnet, daß** die Dosierung mittels einer elektrostatischen Ladung der Substanz durch ein erstes Felderzeugungselement (23) erreicht wird, wodurch die Substanz zu einem Beförderungselement befördert wird, welches die Substanz anzieht und welches die Substanz dann mittels der Einwirkung eines weiteren elektrischen Felds, welches durch ein zweites Felderzeugungselement (32) erzeugt wird, abgegeben wird.

2. Vorrichtung nach Anspruch 1, **gekennzeichnet durch** das Beförderungselement, welches die zu dosierende Substanz befördert, wobei dieses aus einer Dosierungstrommel (24) besteht, welche bei einer Drehung mittels eines eingebauten Motors (26) die Substanz mittels der Einwirkung des zusätzlichen elektrischen Felds, welches **durch** das zweite Felderzeugungselement (32) erzeugt wird, abgibt.

3. Vorrichtung nach Anspruch 1 oder 2, **dadurch gekennzeichnet, daß** die Substanz mittels einer Kombination des zusätzlichen elektrischen Felds und des Luftstroms, welcher vom Einsaugen herrührt, von der Dosierungstrommel (24) in der Vorrichtung abgegeben wird.

4. Vorrichtung nach Anspruch 1 oder 2, **dadurch gekennzeichnet, daß** das dadurch geschaffene Inhalationsgerät ferner eine Einsaugsteuerung aufweist, wodurch keine Substanz von der Dosierungstrommel (24) abgegeben wird, bis ein Luftstrom mittels eines Luftstrommessers (15) erfaßt wird.

5. Vorrichtung nach Anspruch 1 oder 2, **dadurch gekennzeichnet, daß** eine Substanz mittels eines elektronischen Siebs (28) dosiert und gemessen wird, während gleichzeitig die Luftmenge mittels des Luftstrommessers (15) gemessen wird, wobei dies eine flexible und mit hoher Genauigkeit meßbare Dosierung des Pulvers liefert und eine hohe Flexibilität im Hinblick auf den Einsaugvorgang des Patienten ermöglicht.

6. Vorrichtung nach Anspruch 1 oder 2, **dadurch gekennzeichnet, daß** das geschaffene Inhalationsgerät ferner mit einer Behandlungseinrichtung versehen ist, welche aus einem Beschleunigungselement (29), einem Deionisierungselement (30) und einem Verteilerelement (16) besteht, welche dadurch in einem patientenfreundliches Düsenstück aufgenommen sind.

7. Vorrichtung nach Anspruch 1 oder 2, **dadurch gekennzeichnet, daß** das geschaffene Inhalationsgerät mit einem Mikroprozessor (42) versehen ist, welcher zur Steuerung von Dosierung, Strömen, Zeit, Anzahl der Dosen, Begrenzungen, Sicherheitscodes und ähnlichem programmiert werden kann, wobei dies ferner eine schnellere Folgephase der Patientenverwendung der Substanz bzw. der Substanzen während einer Zeitspanne ermöglicht, welche beispielsweise Überdosierung, fehlerhafte Verwendung oder Tendenzen anzeigt.

8. Vorrichtung nach Anspruch 1 oder 2, **dadurch gekennzeichnet, daß** das geschaffene Inhaliergerät mit einer Funktionsanzeigevorrichtung (43) versehen ist, welche beispielsweise mittels Schall, Lichtsignalen und einfachen Texten in dem Fall einer Gefahr von Überdosierung oder fehlerhafter Verwendung eine Warnung ausgibt und ferner eine Freigabe im Fall einer richtig abgegebenen und durch den Patienten aufgenommenen Dosis erteilt.

9. Vorrichtung nach Anspruch 1 oder 2, **dadurch gekennzeichnet, daß** das Inhalationsgerät derart gestaltet ist, daß dessen Aufnahme einer möglichen Patrone zum Laden des Inhalationsgeräts mit der erwünschten Substanz zu ermöglichen, wodurch das Inhalationsgerät mit einer oder mehreren Substanzen einfach ladbar und ferner nachladbar gemacht wird.

10. Vorrichtung nach Anspruch 1 oder 2, **dadurch gekennzeichnet, daß** die pulvrige Substanz mittels Reibungsladung bzw. induktiver Ladung elektrostatisch auf ein geeignetes Potential geladen wird, wenn dies für die Eigenschaften des verwendeten Pulvers besser geeignet ist.

11. Vorrichtung nach Anspruch 1 oder 2, **dadurch gekennzeichnet, daß** das Inhalationsgerät mit der Person, welche das Einsaugen durchführt, elektrisch verbunden ist, wodurch die Gefahr, daß sich Pulver im Mund und in den oberen Luftwegen absetzt, vermindert wird.

12. Vorrichtung nach Anspruch 1 oder 2, **dadurch gekennzeichnet, daß** die pulvrigen chemischen und biologischen Substanzen, welche dosiert werden, derart vorbereitet werden, daß deren elektrische Eigenschaften für die elektrische Dosierung geeignet sind.

13. Vorrichtung nach Anspruch 1 oder 2, **dadurch gekennzeichnet, daß** die elektrischen Merkmale des Pulvers durch die Zugabe von Trägersubstanzen oder durch Oberflächenbehandlung des Pulvers optimiert werden und daß eine geeignete Teilchengröße und Teilchenstruktur verwendet wird.

## Revendications

1. Dispositif pour inhalateur comprenant une source d'énergie pour la création de potentiels électriques appropriés pour l'administration de substances chimiques et biologiques de préférence en poudre, **caractérisé en ce que** le dosage est effectué au moyen d'une charge électrostatique de la substance via un premier élément de génération de champ (23) de sorte que la substance est transmise à un élément de transmission, qui attire la dite substance, et la substance est ensuite émise à partir de ce dernier élément sous l'influence d'un autre champ électrique engendré par un deuxième élément de génération de champ (32).

2. Dispositif selon la revendication 1, **caractérisé en ce que** l'élément de transmission, qui transmet la substance à doser, consiste en un tambour de dosage (24) qui émet, pendant la rotation entraînée par un moteur incorporé (26), la substance sous l'influence du champ électrique additionnel engendré par le deuxième élément de génération de champ (32).

3. Dispositif selon la revendication 1 ou 2, **caractérisé en ce que** la substance est émise à partir du tambour de dosage (24) du dispositif au moyen d'une combinaison du champ électrique additionnel et du flux d'air résultant de l'inspiration.

4. Dispositif selon la revendication 1 ou 2, **caractérisé en ce que** l'inhalateur ainsi créé est additionnellement commandé par l'inspiration, de sorte qu'aucune substance n'est émise à partir du tambour de dosage (24) jusqu'à ce qu'un flux d'air ait été détecté au moyen d'un débitmètre d'air (15).

5. Dispositif selon la revendication 1 ou 2, **caractérisé en ce que** la substance est dosée et mesurée au moyen d'une grille électronique (28) et en même temps la quantité d'air est mesurée au moyen du débitmètre d'air (15), ce qui assure un dosage souple et mesurable avec une grande précision de la poudre et permet une grande souplesse en ce qui concerne le processus d'inspiration du patient.

6. Dispositif selon la revendication 1 ou 2, **caractérisé en ce que** l'inhalateur créé comprend en outre un moyen de traitement consistant en un élément d'accélération (29), un élément de désionisation (30) et un élément de diffusion (16), qui sont ainsi inclus dans un élément d'embouchure commode pour le patient.

7. Dispositif selon la revendication 1 ou 2, **caractérisé en ce que** l'inhalateur créé comprend un microprocesseur (42) qui peut être programmé pour la commande du dosage, des débits, du temps, du nombre de doses, des limites, des codes de sécurité, etc., et qui permet en outre un suivi poussé de la consommation de la substance ou des substances par le patient pendant une certaine période afin de signaler par exemple un surdosage, un usage erroné ou des tendances.

8. Dispositif selon la revendication 1 ou 2, **caractérisé en ce que** l'inhalateur créé comprend un indicateur de fonction (43) qui prévient, par exemple au moyen de signaux sonores, de signaux lumineux et de textes ordinaires, en cas de risque de surdosage ou d'usage erroné, et qui donne également un accord dans le cas d'une dose correctement émise et reçue par le patient.

9. Dispositif selon la revendication 1 ou 2, **caractérisé en ce que** l'inhalateur est agencé de sorte qu'il puisse recevoir une cartouche acceptable pour le chargement de l'inhalateur avec la substance désirée, l'inhalateur étant ainsi rendu facilement chargeable et également rechargeable avec une ou plusieurs substances.

10. Dispositif selon la revendication 1 ou 2, **caractérisé en ce que** la substance en poudre est chargée électrostatiquement par charge triboélectrique ou charge inductive à un potentiel approprié lorsque cela convient mieux pour les caractéristiques de la poudre utilisée.

11. Dispositif selon la revendication 1 ou 2, **caractérisé en ce que** l'inhalateur est électriquement connecté à la personne effectuant l'inspiration, ce qui réduit le risque de dépôt de poudre dans la bouche et dans les voies respiratoires supérieures.

12. Dispositif selon la revendication 1 ou 2, **caractérisé en ce que** les substances chimiques et biologiques en poudre qui sont dosées sont préparées de sorte que leurs caractéristiques électriques conviennent pour le dosage électrique.

13. Dispositif selon la revendication 1 ou 2, **caractérisé en ce que** les caractéristiques électriques de la poudre sont optimisées par l'addition de substances porteuses, ou par le traitement de surface de la poudre, et **en ce qu'**on utilise une dimension de particule et une structure de particule correctes.
